# DEMANDE DE BREVET EUROPEEN

(11) **EP 2 092 933 A1**
(43) Date de publication de la demande: **26.08.2009**
(21) Numéro de dépôt: 09153337.2
(22) Date de dépôt: 20.02.2009
(51) Int. Cl.: A61K 8/41, A61Q 5/10

(54) **Utilisation de polymères cationiques particuliers dans une composition de teinture et associés à un agent chélatant comme agents anti-oxydants ou anti-radicalaires**

(30) Priorité: 22.02.2008 FR 0851167
(71) Demandeur: L'Oréal, 75008 Paris (FR)
(72) Inventeur: Ascione, Jean-Marc, 75003, PARIS (FR)
(74) Mandataire: Fevrier, Murielle Françoise E.

(57) **Abrégé**

La présente invention concerne l'utilisation d'un ou plusieurs polymères cationiques et/ou amphotères de densité de charge cationique supérieure ou égale à 5 meq/gramme, dans une composition comprenant un ou plusieurs colorants d'oxydation et un ou plusieurs agents séquestrants pour un effet radicalaire et/ou antioxydant.

L'invention permet d'obtenir une plus faible dégradation des cheveux lors de l'application d'une composition oxydante.

## Description

La présente invention concerne l'utilisation dans des compositions capillaires de coloration d'oxydation de polymères cationiques particuliers comme agents anti-oxydants ou anti-radicalaires (ou anti radicaux libres).

Il est connu d'utiliser des agents antioxydants et/ou anti radicalaires dans les compositions cosmétiques pour lutter contre la dégradation des compositions elles mêmes ou des substrats sur lesquels elles sont appliquées. En particulier, il est connu d'utiliser des agents antioxydants ou anti radicaux libres pour diminuer la dégradation des cheveux lors de l'application de compositions capillaires.

Par exemple, la demande de brevet WO 2004/087086 décrit une composition comprenant un composé anti-radicalaire présentant une constante de diffusion inférieure ou égale à 10⁻¹⁴ m²/s. De très nombreux composés anti radicalaires répondant à cette définition sont cités.

Les composés anti oxydants ou anti radicalaires utilisés jusqu'à présent sont des produits parfois peu stables dans les compositions cosmétiques ou sur les substrats sur lesquels ils sont appliqués. Ces produits présentent par ailleurs le plus souvent peu d'affinité pour ces substrats, notamment pour les matières kératiniques telles que les cheveux. Ils sont alors facilement éliminés ce qui réduits leur capacité à limiter la dégradation du substrat. Leur impact cosmétique est donc le plus souvent médiocre voire parfois défavorable.

Enfin, leur efficacité est souvent limitée en particulier dans des compositions oxydantes.

Le but de la présente invention est de mettre à disposition de nouveaux systèmes antioxydants ou anti-radicaux libres ne présentant pas les inconvénients de la technique antérieure. En particulier, le but de l'invention est de fournir des antioxydants ou anti radicalaires très efficaces (même en milieu oxydant), faciles à mettre en oeuvre dans les compositions cosmétiques, stables, présentant une bonne affinité pour les substrats tels que les cheveux, ayant un bon effet cosmétique.

Ainsi, la présente invention a pour objet l'utilisation d'un ou plusieurs polymères cationiques et/ou amphotères de densité de charge cationique supérieure ou égale à 5 meq/gramme en tant qu'agent radicalaire et/ou antioxydant, dans une composition comprenant un ou plusieurs colorants d'oxydation et un ou plusieurs agents séquestrants.

L'utilisation d'une telle composition permet de lutter efficacement contre l'effet destructeur des radicaux hydroxyles OH°. Cette action sur les radicaux hydroxyles est intéressante dans les milieux oxydants utilisés en coloration d'oxydation des cheveux car ces radicaux sont souvent responsables de la dégradation des fibres kératiniques. Cette utilisation permet notamment d'améliorer l'effet anti radicalaire et/ou antioxydant, lors du mélange de cette composition avec un agent oxydant, tel que le peroxyde d'hydrogène.

Comme indiqué précédemment les polymères utiles dans l'invention possèdent une densité de charge cationique supérieure ou égale à 5 meq/gramme.Cette densité de charge peut se déterminer expérimentalement par la méthode Kjeldahl ou par calcul à partir de la structure du polymère.

Ces polymères peuvent être cationiques ou amphotères

### Polymères cationiques :

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire en nombre comprise entre 500 et 5.10⁶ environ, et de préférence comprise entre 10³ et 3.10⁶ environ.

Les polymères cationiques de l'invention sont de préférence choisis parmi :
(1)les cyclopolymères de méthyl diallyl amine ou de diallyl diméthyl ammonium tels que les homopolymères ou les copolymères comportant comme constituant principal de la chaîne des motifs répondant aux formules (I) ou (I') : 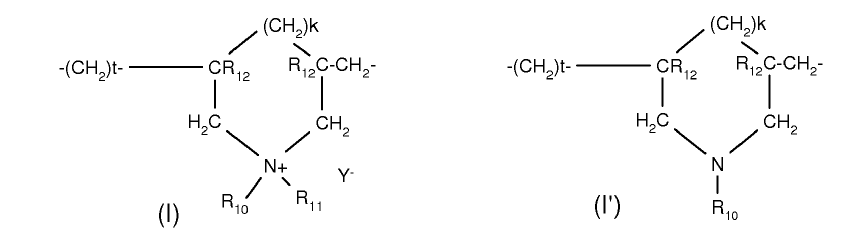 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement les homopolymères de chlorure de diallyldiméthylammonium tels que le Merquat 100 proposé par la société NALCO.et les copolymères d'acrylamide et de chlorure de diallyldiméthylammonium tels que le merquat 550 proposé par la société NALCO.
(2)les polymères de diammonium quaternaire contenant des motifs récurents répondant à la formule (II) : 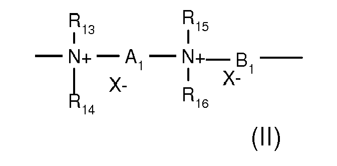 formule (II) dans laquelle :
   R₁₃, R₁₄, R₁₅ et R₁₆, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH₂)ₙ-CO-D-OC-(CH₂)ₙ-
   dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- ; De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 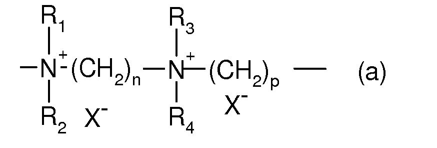 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 6 environ et, X⁻ est un anion dérivé d'un acide minéral ou organique.
   Un composé de formule (a) particulièrement préféré est celui pour lequel R₁, R₂, R₃ et R₄ représentent un groupe méthyle et n = 3, p= 6 et X = Cl, c'est-à-dire le chlorure d'hexadimethrine dénommé "Hexadimethrine chloride" selon la nomenclature INCI (CTFA), propsé par la société CHIMEX sous l'appelation Mexomer PO.
(3)les polymères de polyammonium quaternaires constitués de motifs de formule (III): 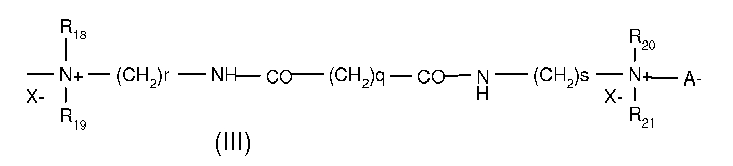 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "MIRAPOL A 15", "MIRAPOL® AD1", "MIRAPOL® AZ1" et "MIRAPOL® 175" vendus par la société MIRANOL.
(4)D'autres polymères cationiques utilisables dans le cadre de l'invention sont des polyalkylèneimines, en particulier des polyéthylèneimines.
   Polymères amphotères.
   Les polymères amphotères utilisables conformément à l'invention peuvent être choisis parmi les polymères comportant des motifs A et B répartis statistiquement dans la chaîne polymère où A désigne un motif dérivant d'un monomère comportant au moins un atome d'azote basique et B désigne un motif dérivant d'un monomère acide comportant un ou plusieurs groupements carboxyliques ou sulfoniques ou bien A et B peuvent désigner des groupements dérivant de monomères zwittérioniques de carboxybétaïnes ou de sulfobétaïnes ;
   A et B peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien A et B font partie d'une chaîne d'un polymère à motif éthylène □.□-dicarboxylique dont l'un des groupements carboxyliques a été amené à réagir avec une polyamine comportant un ou plusieurs groupements amine primaire ou secondaire.
   Les polymères amphotères répondant à la définition donnée ci-dessus plus particulièrement préférés sont choisis parmi les:
(5) les polymères dérivés de diallyldialkylammonium et d'au moins un monomère anionique tels que les polymères comportant environ 60 à environ 99% en poids d'unités dérivées d'un monomère de diallyldialkylammonium quaternaire dans lequel les groupements alkyle sont choisis indépendamment parmi les groupements alkyle ayant 1 à 18 atomes de carbone et dans lequel l'anion est dérivé d'un acide ayant une constante d'ionisation supérieure à 10⁻¹³ et 1 à 40% en poids de ce polymère, d'un monomère anionique choisi parmi les acides acrylique ou méthacrylique, le poids moléculaire de ce polymère étant compris entre environ 50.000 et 10.000.000 déterminé par chromatographie par perméation de gel. De tels polymères sont décrits dans la demande EP-A-269243.

Les polymères préférés sont entre autres les polymères comportant des groupements alkyle choisis parmi les groupements ayant 1 à 4 atomes de carbone et plus particulièrement des groupements méthyle, éthyle.

Parmi ces polymères, les copolymères de chlorure de diméthyldiallylammonium ou de diéthyldiallylammonium et d'acide acrylique sont particulièrement préférés. Ces polymères sont par exemple vendus sous les dénominations "MERQUAT 280" et "MERQUAT 295" par la société MERCK.

On peut également utiliser les terpolymères de chlorure de diméthyldiallylammonium/ acide acrylique/acrylamide vendu sous la dénomination "MERQUAT PLUS 3330" par la société MERCK.

Selon l'invention, on peut également utiliser les polymères amphotères sous forme de latex ou de pseudolatex, c'est-à-dire sous forme d'une dispersion aqueuse de particules de polymères insolubles.

Selon l'invention, on peut utiliser plus particulièrement le Mexomer PO , le Merquat 100, le Merquat 280 ou le Mirapol A15.

Le ou les polymères cationiques ou amphotères de densité de charge cationique supérieure ou égale à 5 meq/gramme sont généralement présents dans la composition tinctoriale dans des quantités comprises allant de 0.00001 % à 10% en poids par rapport au poids de la composition.

### Colorants d'oxydation.

Les compositions de coloration d'oxydation comprennent un ou plusieurs colorants d'oxydation. En général ces compositions contiennent une ou plusieurs bases d'oxydation, et éventuellement un ou plusieurs coupleurs.

A titre de bases d'oxydation classiquement utilisées, on peut citer les ortho- et paraphénylènediamines, les bases doubles, les ortho- et para- aminophénols, les bases hétérocycliques suivantes ainsi que leurs sels d'addition avec un acide.

On peut citer en particulier :
(A) les paraphénylènediamines de formule (IV) suivante et leurs sels d'addition avec un acide : 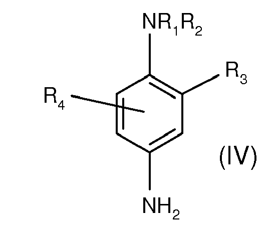 dans laquelle :
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.
   Parmi les groupements azotés de la formule (IV) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (IV) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (IV) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
-(B) Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales prêtes à l'emploi conformes à l'invention, on peut notamment citer les composés répondant à la formule (V) suivante, et leurs sels d'addition avec un acide : 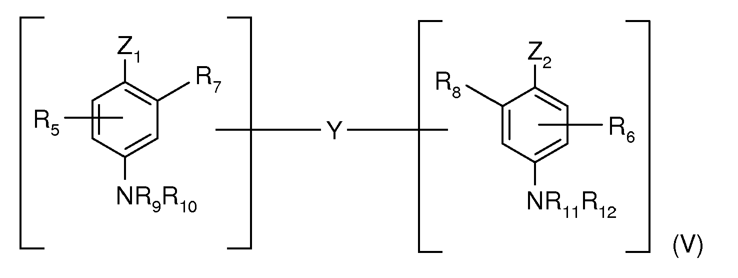 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄
   ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (V) ne comportent qu'un seul bras de liaison Y par molécule.
   Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (V) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (V), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (C) les para-aminophénols répondant à la formule (VI) suivante, et leurs sels d'addition avec un acide : 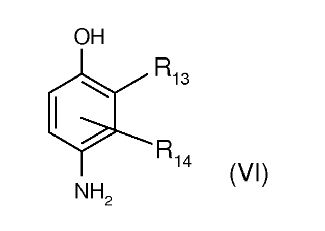 dans laquelle :
   R₁₃ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).
   Parmi les para-aminophénols de formule (VI) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (D) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
-(E) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

A titre de dérivés pyrazoliques, on peut aussi citer les diamino-N,N-dihydropyrazopyrazolones et notamment celles décrites dans la demande FR 2 886 136 telles que les composés suivants et leurs sels d'addition.

Parmi ces composés, les préférés sont les :
2,3-Diamino-6,7-dihydro-1H,5H-pyrazolo[1,2-a]pyrazol-1-one,
2-Amino-3-éthylamino-6,7-dihydro-1H,5H-pyrazolo[1,2-a]pyrazol-1-one,
2-Amino-3-isopropylamino-6,7-dihydro-1H,5H-pyrazolo[1,2-a]pyrazol-1-one,
2-Amino-3-(pyrrolidin-1-yl)-6,7-dihydro-1H,5H-pyrazolo[1,2-a]pyrazol-1-one,
4,5-diamino-1,2-diméthyl-1,2-dihydro-pyrazol-3-one,
4,5-diamino-11,2-diéthyl-,2-dihydro-pyrazol-3-one,
4,5-diamino-1,2-di-(2-hydroxyéthyl)-1,2-dihydro-pyrazol-3-one,
2-amino-3-(2-hydroxyéthyl)amino-6,7-dihydro-1H,5H-pyrazolo[1,2-a]pyrazol-1-one,
2-amino-3-diméthylamino-6,7-dihydro-1H,5H-pyrazolo[1,2-a]pyrazol-1-one,
2,3-diamino-5,6,7,8-tétrahydro-1H,6H-pyridazino[1,2-a]pyrazol-1-one,
4-Amino-1,2-diethyl-5-(pyrrolidin-1-yl)-1,2-dihydro-pyrazol-3-one,
4-Amino-5-(3-dimethylamino-pyrrolidin-1-yl)-1,2-diethyl-1,2-dihydro-pyrazol-3-one,
2,3-diamino-6-hydroxy-6,7-dihydro-1H,5H-pyrazolo[1,2-a]pyrazol-1-one,

A titre de bases hétérocycliques, on utilisera préférentiellement le 4,5-diamino 1-(β-hydroxyéthyl) pyrazole, la 2,3-diamino-6,7-dihydro-1H,5H-pyrazolo[1,2-a]pyrazol-1-one et leurs sels d'addition.

Le ou les coupleurs utilisables sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

La ou les bases d'oxydation et le ou les coupleurs sont en général présents chacun en une quantité allant de 0,0005 à 12% en poids, de préférence en une quantité allant de 0,01 à 8% en poids, par rapport au poids total de la composition.

D'une manière générale, les sels d'addition avec un acide des bases d'oxydation et des coupleurs sont notamment choisis parmi les chlorhydrates, les bromhydrates, les sulfates et les tartrates, les lactates et les acétates.

Ces compositions peuvent aussi contenir un ou plusieurs colorants directs pouvant notamment être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Le ou les colorants directs représentent de préférence de 0,001 à 20% en poids environ du poids total de la composition tinctorialeet encore plus préférentiellement de 0,005 à 10% en poids environ

### Agents séquestrants

Par « agent séquestrant », on entend dans la présente invention un composé capable de se combiner aux métaux de transition.

Au sens de la présente invention, on entend par "métal de transition", un métal comportant une sous-couche d incomplète, plus particulièrement à l'état d'oxydation II, tel que le cobalt (Co²⁺), le fer (Fe²⁺), le manganèse (Mn²⁺), le zinc (Zn²⁺) et le cuivre (Cu²⁺).

De manière générale les agents séquestrants ou agents chélatants sont décrits dans l'Encyclopédie « KIRK-OTHMER Encyclopedia of Chemical Technology John Wiley and sons Vol 5 pages 708 à 739.

Parmi les agents séquestrants utilisables dans la composition de l'invention, on peut citer en particulier :
- les acides tri ou tétracarboxyliques ou leurs sels comme par exemple l'acide méthylglycine diacétique, l'acide N-lauroyl-N,N',N'-tri-acétique éthylènediamine, l'acide iminodisuccinique, l'acide N,N-dicarboxyméthyl L-glutamique l'acide éthylènediamine-N,N'-dissucinique,l'acide éthylène diamine tétracétique (EDTA) ;l'acide citrique,
- les dérivés phosphoniques tels que l'acide hexaméthylène diamine tétra(méthylène phosphonique), l'acide éthylène diamine tétra(méthylène phosphonique), l'acide 1-hydroxyéthylidène 1,1-diphosphonique, l'acide aminotri(méthylène phosphonique), l'acide diéthylène-triamine penta(méthylène phosphonique), et leurs sels et notamment leurs sels de sodium comme le sel pentasodique de l'acide éthylènediamine tétra(méthylène phosphonique)
- , les dendrimères à activité chélatante ;
- les protéines comme la spermine, la spermidine, la transférine, la ferritine ;
- les acides mono ou di carboxyliques carboxyliques ou leurs sels comme l'acide phytique,l'acide malique, l'acide nitrilo-acétique, l'acide fumarique, l'acide tartrique, l'acide succinique, l'acide oxalique, l'acide mucique ;
- la desferrioxamine mesylate ;
et les mélanges de ces séquestrants

Les acides carboxylique sont préférés, notamment les acides tri ou tétracarboxyliques.. Préférentiellement ces acides comportent au moins un atome d'azote dans leur structure.

Le ou les agents séquestrants représentent en général de 0,00001 à 20% en poids du poids total de la composition.

Les compositions de l'invention peuvent comprendre un ou plusieurs agents oxydants.

A titre d'agents oxydants pouvant être utilisés, on peut citer le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases et leurs cofacteurs éventuels tels que l'acide urique pour les uricases. Le peroxyde d'hydrogène est particulièrement préféré.

Le pH de la composition est généralement compris entre les valeurs 2 et 14. Il est de préférence compris entre 5 et 13.

Selon une variante, la composition cosmétique présente un pH alcalin entre 7 et 14, de préférence entre 8 et 12. A titre d'agents alcalins pouvant être présents, on peut citer l'ammoniaque, les carbonates alcalins, le carbonate d'ammonium, les acides aminés et en particulier les acides aminés basiques tels que la lysine ou l'arginine, les alcanolamines telles que les mono-, di- et triéthanolamines ainsi que leurs dérivés, les hydroxydes de sodium ou de potassium et les composés de formule suivante : 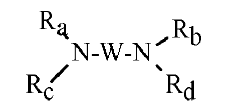 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La quantité d'eau présente dans la composition cosmétique est en général comprise entre 5 et 95 %, de préférence 20 et 90 %, mieux encore 30 et 80 %.

La composition tinctoriale selon l'invention peut se présenter sous des formes diverses, telles que sous forme de liquides, de crèmes, de gels, ou sous toute autre forme appropriée pour être appliquer sur les fibres kératiniques, et notamment des cheveux humains.

Le milieu approprié pour la teinture (ou support) de la composition utile à l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% en poids et, de préférence, entre environ 2 et 10% en poids par rapport au poids total de la composition.

La composition cosmétique utile dans l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères autres que les polymères cationiques ou amphotères de l'invention, des agents épaississants minéraux ou organiques, d'autres agents antioxydants ou anti-radicaux libres, des agents de pénétration, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20% en poids par rapport au poids de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La couleur est révélée au moment de l'emploi par mélange d'une composition tinctoriale telle que décrite ci-dessus sans agents oxydants avec une composition oxydante comprenant un ou plusieurs agents oxydants.

A titre d'agents oxydants pouvant être utilisés, on peut citer le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases et leurs cofacteurs éventuels tels que l'acide urique pour les uricases. Le peroxyde d'hydrogène est particulièrement préféré.

Le mélange peut être extemporané.

On applique alors ce mélange sur les cheveux pendant un temps suffisant pour développer la coloration désirée(habituellement de 5 à 60 minutes), puis on rince, on lave éventuellement au shampooing, on rince à nouveau et on sèche.

On peut aussi appliquer directement les compositions tinctoriales sur les cheveux simultanément ou de manière décalée avec dans dernier cas possibilité d'un rinçage intermédiaire.

L'exemple qui suit est destiné à illustrer l'invention sans pour autant en limiter la portée.

### EXEMPLE

Composition tinctoriale.

| | CONCENTRATION EN G |
|---|---|
| ACIDE DIETHYLENE TRIAMINE PENTACETIQUE, SEL PENTASODIQUE EN SOLUTION AQUEUSE A 40% | 2 |
| THIOLACTATE D'AMMONIUM EN SOLUTION AQUEUSE A 58 % (50 % EN ACIDE THIOLACTIQUE) | 0,80 |
| AMMONIAQUE (CONCENTRATION DE REFERENCE 20% EN AMMONIAC) | 10 |
| MONOETHANOLAMINE PURE | 1,35 |
| SILICE PYROGENEE A CARACTERE HYDROPHOBE | 1,20 |
| 1-BETA-HYDROXYETHYLOXY-2,4-DIAMINO-BENZENE DICHLORHYDRATE | 0,15 |
| 2-METHYL-1,3-DIHYDROXYBENZENE (2-METHYLRESORCINOL) | 0,81 |
| OXYDE DE TITANE (ANATASE NON TRAITE) ENROBE DE POLY DIMETHYLSILOXANE (98/2) (CI: 77891) | 0,15 |
| SULFATE DE N,N-BIS(2-HYDROXYETHYL)-P-PHENYLENEDIAMINE, 1 H₂O | 0,21 |
| 1,3-DIHYDROXYBENZENE (RESORCINOL) | 1,29 |
| 1-HYDROXY-3-AMINO-BENZENE | 0,33 |
| 1,4-DIAMINO-BENZENE | 1,84 |
| DISTEARATE DE GLYCOL | 2 |
| ALCOOL CETYLSTEARYLIQUE (C₁₆/C₁₈ 50/50) | 11,50 |
| PARFUM | 0,60 |
| POLYCONDENSAT TETRAMETHYL HEXAMETHYLENEDIAMINE/DICHLORO 1,3-PROPYLENE EN SOLUTION AQUEUSE | 4 |
| POLYMERE CARBOXYVINYLIQUE SYNTHETISE DANS LE MELANGE ACETATE D'ETHYLE/CYCLOHEXANE | 0,60 |
| PROPYLENE GLYCOL | 7 |
| ACIDE LAURIQUE NATUREL | 3 |
| ALCOOL LAURIQUE OXYETHYLENE (12 OE) | 7 |
| ALCOOL DECYLIQUE OXYETHYLENE (3 OE) | 10 |
| ALCOOL OLEOCETYLIQUE OXYETHYLENE (30 OE) | 4 |
| EAU DESIONISEE | QSP 100G |

Cette composition est mélangée poids pour poids avec un oxydant à pH2.3 comprenant 9% de peroxyde d'hydrogène.On applique 30 mn le mélange obtenu sur des cheveux gris à 90% de blancs. Après rinçage et séchage les cheveux sont teints dans une nuance châtain.

Les cheveux sont doux et peu dégradés.

## Revendications

1. Utilisation d'un ou plusieurs polymères cationiques et/ou amphotères de densité de charge cationique supérieure ou égale à 5 meq/gramme , en tant agent anti-radicalire et/ou antioxydant dans une composition comprenant un ou plusieurs colorants d'oxydation et un ou plusieurs agents séquestrants.

2. Utilisation selon la revendication 1 dans laquelle le polymère cationique est choisi parmi les cyclopolymères de méthyl diallyl amine ou de diallyl diméthyl ammonium tels que les homopolymères ou les copolymères comportant comme constituant principal de la chaîne des motifs répondant aux formules (I) ou (I') : 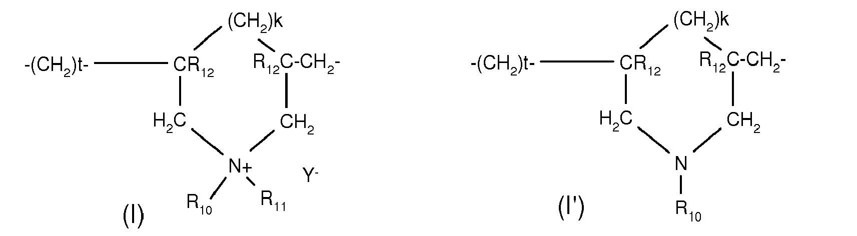 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.

3. Utilisation selon la revendication 2 dans laquelle le polymère cationique est choisi parmi les homopolymères de chlorure de diallyldiméthylammonium et les copolymères d'acrylamide et de chlorure de diallyldiméthylammonium.

4. Utilisation selon la revendication 1 dans laquelle le polymère cationique est choisi parmi les polymères de diammonium quaternaire contenant des motifs récurents répondant à la formule (II) : 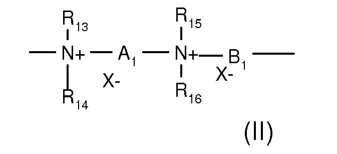 formule (II) dans laquelle :
R₁₃, R₁₄, R₁₅ et R₁₆, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R17-D ou -CO-NH-R₁₇-D où R₁₇ est un alkylène et D un groupement ammonium quaternaire ;
A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A₁, R₁₃ et R₁₅ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH₂)ₙ-CO-D-OC-(CH₂)ₙ-
dans lequel D désigne :
a) un reste de glycol de formule : -O-Z-O-, où Z désigne un radical hydrocarboné linéaire ou ramifié ou un groupement répondant à l'une des formules suivantes :
-(CH₂-CH₂-O)ₓ-CH₂-CH₂-
-[CH₂-CH(CH₃)-O]_{y}-CH₂-CH(CH₃)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH₂-CH₂-S-S-CH₂-CH₂- ;
d) un groupement uréylène de formule : -NH-CO-NH- ;

5. Utilisation selon la revendication 4 dans laquelle le polymère cationique est choisi parmi les polymères qui sont constitués de motifs récurrents répondant à la formule : 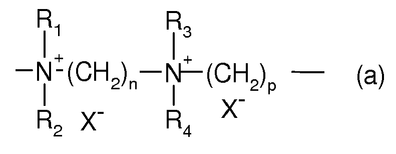 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 6 environ et, X⁻ est un anion dérivé d'un acide minéral ou organique.

6. Utilisation selon la revendication 5 dans laquelle R₁, R₂, R₃ et R₄ représentent un groupe méthyle et n = 3, p= 6 et X = Cl

7. Utilisation selon la revendication 1 dans laquelle le polymère cationique est choisi parmi les polymères les polymères de polyammonium quaternaires constitués de motifs de formule (III): 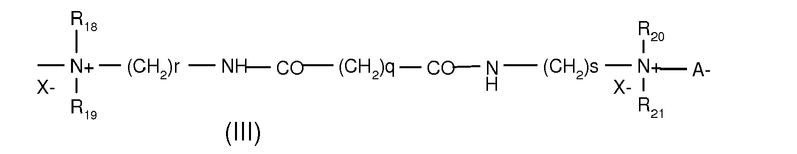 formule dans laquelle :
R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH₂-CH₂-O-CH₂-CH₂-.

8. Utilisation selon la revendication 1 dans laquelle le polymère amphotère est choisi parmi les polymères dérivés de diallyldialkylammonium et d'au moins un monomère anionique

9. Utilisation selon l'une quelconque des revendications précédentes dans laquelle le
ou les polymères cationiques ou amphotères de densité de charge cationique supérieure
ou égale à 5 meq/gramme sont présents dans la composition tinctoriale dans des quantités comprises allant de 0.00001% à 10% en poids par rapport au poids de la composition.

10. Utilisation selon l'une quelconque des revendications précédentes dans laquelle les agents séquestrants sont choisis parmi :
- les acides tri ou tétracarboxyliques ou leurs sels
- les dérivés phosphoniques et leurs sels
- les dendrimères;
- les protéines comme
- les acides mono ou di carboxyliques carboxyliques ou leurs sels
- la desferrioxamine mesylate ;
et les mélanges de ces séquestrants

11. Utilisation selon la revendication précédente **caractérisée en ce que** le ou les agents séquestrants sont choisis parmi les acides tri ou tétracarboxyliques, comprenant de façon optionnelle au moins un atome d'azote dans leur structure.

12. Utilisation selon l'une quelconque des revendications précédentes **caractérisée en ce que** la composition comprend un ou plusieurs agents oxydants, de préférence choisi parmi le peroxyde d'hydrogène, le peroxyde d'urée, les bromates de métaux alcalins, les persels, les peracides et les enzymes oxydases.

13. Utilisation selon l'une quelconque des revendications précédentes dans laquelle la composition présente un pH compris entre 7 et 14, de préférence entre 8 et 12.
